(19) Europäisches Patentamt / European Patent Office / Office européen des brevets

(11) **EP 2 150 176 B1**

(12) **EUROPEAN PATENT SPECIFICATION**

(45) Date of publication and mention
of the grant of the patent:
**27.04.2016 Bulletin 2016/17**

(21) Application number: **07748075.4**

(22) Date of filing: **27.04.2007**

(51) Int Cl.:
*A61B 5/145* *(2006.01)*        *A61N 1/365* *(2006.01)*
*G01N 33/49* *(2006.01)*        *A61B 5/1455* *(2006.01)*
*A61B 5/1459* *(2006.01)*        *A61N 1/05* *(2006.01)*
*G01N 33/543* *(2006.01)*        *G01N 21/77* *(2006.01)*
*G01N 21/64* *(2006.01)*

(86) International application number:
**PCT/SE2007/000410**

(87) International publication number:
**WO 2008/133551 (06.11.2008 Gazette 2008/45)**

(54) **IMPLANTABLE CONCENTRATION SENSOR AND DEVICE**

IMPLANTIERBARER KONZENTRATIONSSENSOR UND VORRICHTUNG

DÉTECTEUR DE CONCENTRATION IMPLANTABLE ET DISPOSITIF

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**10.02.2010 Bulletin 2010/06**

(73) Proprietor: **St. Jude Medical AB**
**175 84 Järfälla (SE)**

(72) Inventor: **DOWLING, Kenneth**
**S-197 91 Bro (SE)**

(74) Representative: **Sjölander, Henrik et al**
**Aros Patent AB**
**P.O. Box 1544**
**751 45 Uppsala (SE)**

(56) References cited:
**EP-A2- 0 252 578          WO-A1-00/13003**
**WO-A1-93/12710          US-A- 4 399 820**
**US-A- 4 476 870          US-A- 4 968 632**
**US-A- 5 490 323          US-A- 5 498 549**
**US-A- 5 517 313          US-A- 5 882 936**
**US-A1- 2004 152 187     US-A1- 2005 075 673**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the Invention

The present invention generally relates to the field of implantable medical devices. More specifically, the present invention relates to a sensor, a medical lead, an implantable device and a method for determining the concentration of a substance and/or molecule in blood or tissue of a patient, in particular for determining the concentration of substances and or molecules such as oxygen, carbon dioxide, molecules representative of a PH value, glucose, urea, ammonia, lactose, hormones and insulin.

Background Art

Many substances and/or molecules are comprised in blood, which works as a transporting medium to either supply these substances and/or molecules to organs of a human body or to remove them. As examples, blood can supply molecules such as oxygen or nutrients such as glucose and amino acids to tissues of organs while it can also remove substances such as urea and carbon dioxide from these organs. The concentrations of these substances and/or molecules in blood or tissue of a human body determine the functioning of the organs and therefore the health of the human body. For instance, the content of oxygen in blood is an important parameter to determine for understanding cardiac and pulmonary function of a patient and is particularly important to monitor for adjusting the frequency of a pacemaker.

Conventional sensors employed to monitor the content of oxygen in blood are based on photo-spectrometric properties of blood, which properties depend on the oxygenation level of blood. In these sensors, such as the sensor disclosed in US 4815469, a light emitting diode is used to illuminate the blood of a patient and a photo-detector is used to detect the light reflected by the blood, the light emitting diode and the photo-detector being separated from the blood by means of an interface, e.g. being embedded in a medical pacing lead. The analysis of the spectrum of the reflected light then results in the content of oxygen in the blood. An alternative to such a measurement is a measurement based on transmitted light where the light source and the photo-detector are positioned on either side of the blood specimen to be analyzed and where the light absorption in hemoglobin is used as an indicator representative of the content of oxygen in blood. One of the requirements for detectors based on reflectance and absorbance measurements is that the interface between the light source and the blood and the interface between the photo-detector and the blood must be unimpeded in the wavelengths used for analysis. However, phenomena such as thrombosis, tissue growth or impeding of blood flow at these interfaces are common and prevent accurate and continuous measurements of the oxygen content of blood such as would be required for pacing or other cardiac therapy. In addition, these optical techniques enhance these phenomena, which cause the measurements to be more and more difficult to implement with time.

Further, another problem with conventional reflectance and absorbance measurements is that the result of the measurement is dependent on variable parameters such as blood flow rate, red blood cell count, white blood cell count, platelet count and other variable factors in the bloodstream since the measurement directly involves the constituents of the blood. In addition, the detectors based on reflectance and absorbance require up to three wavelengths, which is expensive and complicates the detector in terms of design and space management. Thus, there is a need for providing improved methods and devices that would overcome at least some of these problems.

US 4,476,870 discloses an implantable fibre optic probe for monitoring the partial pressure of gaseous oxygen in the blood stream. The probe comprises two optical fibres ending in a section of porous polymer tubing packed with a fluorescent light-excitable dye placed on a porous adsorptive polymer support. A water-impermeable container of high oxygen permeability encloses the polymer support.

US 2004/0152187 discloses a glucose sensor assembly having a first optical fibre connected in one end to a light source and in the other end to a first sensor and a second optical fibre connected in one end to the light source and in the other end to a second sensor. The first sensor comprises a luminescent material encapsulated by an oxygen permeable polymer barrier. The second sensor comprises luminescent material encapsulated by an oxygen permeable polymer barrier and a pocket containing glucose oxidase.

Summary of the Invention

An object of the present invention is to wholly or partly overcome the above disadvantages and drawbacks of the prior art and to provide an improved alternative to the above techniques and prior art.

A further object of the present invention to provide an improved sensor, a medical lead comprising such a sensor, an implantable device connectable to such a medical lead for determining the concentration of a molecule and/or a substance such as oxygen, carbon dioxide, molecules representative of a PH value, glucose, urea, ammonia, lactose, hormones and insulin in blood or tissue of a patient that wholly or partly overcome the above mentioned problems.

Another object of the present invention is to provide an improved sensor that provides accurate and repeatable measurements of a concentration of a molecule and/or a substance such as oxygen, carbon dioxide, molecules representative of a PH value, glucose, urea, ammonia, lactose, hormones and insulin in blood or tissue of a patient.

Hence, an implantable sensor for determining a concentration of a substance and/or molecule in blood or tissue of a patient is provided. The sensor comprises at least one light source adapted to, during measurement sessions, emit light at least at a first wavelength, at least one photo-detector and at least one type of photoluminescent molecules embedded in a carrier selectively permeable to the substance and/or molecule. A part of the carrier is in contact with a region of the patient, which region comprises the substance and/or molecule, and the light source and the photo-detector are optically connected to the carrier. The photoluminescent molecules emit light in response to excitation by the light emitted from the light source, and the substance and/or molecule reacts with the photoluminescent molecules to alter characteristics of the light emitted from the photoluminescent molecules. The photo-detector is adapted to detect the light emitted from the photoluminescent molecules and to provide output signals representative of the characteristics to determine the concentration of the substance and/or molecule.

According to an aspect of the present invention, a medical lead comprising an implantable sensor such as the improved sensor described above is provided. The inventive medical lead may comprise the same additional features as the features that will be described below for the implantable sensor, thus providing similar advantages.

According to another aspect of the present invention, an implantable device connected to a medical lead comprising an implantable sensor such as the improved sensor described above is provided. The implantable device further comprises a blood constituent determining device adapted to receive output signals from the photo- detector in order to determine, based on the received signals, the concentration of the molecules and/or substances being comprised in the blood composition or the tissue of a patient. The inventive implantable device may comprise the same additional features as the features that will be described below for the implantable sensor, thus providing similar advantages.

A method for determining a concentration of a substance and/or a molecule in blood or tissue of a patient is provided. This method comprises a first step of, during measurements sessions, exciting photoluminescent molecules embedded in a carrier selectively permeable to the substance and/or molecule such that the photoluminescent molecules emit light. Further, the method comprises a step of detecting the light emitted from the photoluminescent molecules, and a step of determining the concentration of the substance and/or molecule based on characteristics of the detected light.

The present invention is based on the understanding that photoluminescent molecules can be arranged in an implantable device to determine the concentration of a substance and/or a molecule in blood or tissue of a patient.

The inventive sensor described above is advantageous in that it enables real-time measurements over long time periods. The sensor according to the present invention discloses a low sensitivity to phenomena such as thrombosis, tissue growth or impeding of blood flow since the interface between the sensor and the region to be analyzed is not illuminated, as opposed to prior art sensors in which such phenomena are enhanced by the fact that light has to pass through the interface between the sensor and the blood. Further, as the optical part of the measurement does not take place directly in the blood or the tissue of the patient but in the carrier, the determination of the concentration is not dependent on variable parameters such as blood flow rate, red blood cell count, white blood cell count, patelet count and other variable factors in the bloodstream. Further, as the molecules to be analyzed can penetrate tissue of organs, the sensor may either be arranged directly in contact with the blood of a patient or in the tissue of a patient. In addition, the sensor can be made very compact and cheap.

The implantable sensor may comprise an optical filter arranged at the photo-detector to selectively transmit the light emitted by the photoluminescent molecules to the photo-detector, which is advantageous since the amount of light directly received from the light source by the photo-detector is minimized.

In further embodiments, the carrier of the implantable sensor may be made of a material comprised in the group of silicone rubber (also known as polydimethylsiloxane), organosubstitute silicones such as poly(R-R'-siloxane) wherein R and R' are one of the following {methyl, ethyl, propyl, butyl, "alkyl", "aryl", phenyl and "vinyl"} and not necessarily equal to each other, polyurethane, poly(1-trimethylsily-1-propyne), polystyrene, poly(methylmethacrylate), poly(vinyl chloride), poly(isobutylmethacrylate), poly(2,2,2-trifluoroethylmethacrylate), ethylcellulose, cellulose acetobutyrate, cellulose acetate, gas-permeable polytetrafluoroethylene and thermoplastic polyurethanes and copolymers.

In another embodiment, the light source of the implantable sensor may be a solid state light source, preferably a light emitting diode, which is advantageous since light emitting diodes are small and therefore easily incorporated in the sensor, thus alleviating the requirement on space issues close to the region to be analyzed. Further, the use of light emitting diodes is advantageous since they have a rather long lifetime as compared to most common light sources. Further, light emitting diodes can be selected so that the level of infrared radiation contained in their output power spectra is low in order to minimize the generation of heat by the radiation. In a particular embodiment, the light source may be a light-emitting diode adapted to emit light in the range of 390-1650 nm.

In another embodiment, the photo-detector of the implantable sensor may be a solid state photo-detector, which is advantageous since such detectors present excellent linearity with respect to incident light, have low internal noise, wide spectral response, long life time, and are mechanically resistant, compact and lightweight. In particular embodiments, the photo- detector shall be sensitive in the range of 350-1800 nm, preferably in the range of 350-800 nm, which is advantageous since these are ranges within which most common photoluminescent molecules emit light. In particular embodiments, the photo-detector may be one of the group comprised of a pn photodiode, a pin photodiode, a Schottky photodiode, an avalanche photodiode, a silicon photodiode, a planar InGaAs photodiode, a SiGe-based optoelectronic circuit and a InGaN/GaN multiple quantum well pn junction.

In an embodiment, the implantable sensor may be adapted to determine the concentration of any type of substances and/or molecules being comprised in the blood composition or in the tissue of a patient. In an embodiment, the sensor is adapted to determine the saturation or the concentration of oxygen in the region at which the carrier is arranged. In another embodiment, the sensor is adapted to determine the concentration of carbon dioxide in the region at which the carrier is arranged. In yet another embodiment, the sensor is adapted to determine the PH-value in the region at which the carrier is arranged. In other embodiments, the sensor is adapted to determine the concentration of a substance and/or a molecule of anyone of the group of glucose, urea, ammonia, lactose, hormones or insulin, which molecule or substance is comprised in the region at which the carrier is arranged.

In further embodiments, the photoluminescent molecules may be fluorescent molecules or phosphorescent molecules. Although it would be preferable to use fluorescent molecules as they are generally more stable over time, it is preferable to use phosphorescent molecules as phosphorescence leads to longer timescale, which then facilitates the design of the electronics in the sensor.

In further embodiments, the photoluminescent molecules used in the implantable sensor of the present invention may be one of the group comprised of pyrene, a pyrene derivative (vinylpyrene, methoxypyrene), a polyaromatic carrier, an ionic probe (ruthenium trisbypyridine) and Pd-tetra (4-carboxyphenyl) benzoporphin (Oxyphor). In particular embodiments, the concentration of these photoluminescent molecules is comprised in the range of 20 ppb to 2% w/w, preferentially in the range of 0.1 - 500 ppm, and more preferentially at approximately 5 ppm.

In further embodiments, the inventive implantable sensor may be arranged at the heart of a patient, in arterial blood, in venous blood or in the tissue of a blood-containing organ of a patient. Thus, many arrangements are possible, the requirement being that the carrier is in contact with a region of the patient comprising the molecules and/or substances to be analyzed such that these molecules and/or substances can react with the photoluminescent molecules.

In another embodiment, the light source and the photo-detector are hermetically isolated from the region of the patient.

In further embodiments, one of the characteristics of the light emitted by the photoluminescent molecules, which light may be altered by the reaction between the molecules and/or substances to be analyzed and the photoluminescent molecules, may be the intensity, the lifetime or the wavelength. This is advantageous since this provides different manners of determining the concentration of the molecules and/or substances to be analyzed. In a particular embodiment, it may be advantageous to use the lifetime of the light rather than its intensity since any change in the concentration of the photoluminescent molecules with time will affect the intensity but not the lifetime. Such changes in concentration are possible because of various biological processes.

In an embodiment, the implantable device may comprise an amplifier for amplifying the output signals sent from the photo-detector to the blood constituent determining device, which is advantageous since the output signals of the photo-detector may, in some cases, be weak.

In further embodiments, the lifetime of the light emitted by the photoluminescent molecules may be determined according to different types of measurements. In an embodiment, the implantable device may comprise a pulse generating means connected to the light source to cause the light source to emit light pulses such that the blood constituent determining device, which is adapted to control the photo-detector and the pulse generating means, can perform time resolved measurements for determination of the lifetime. In another embodiment, the implantable device may comprise a frequency modulating means connected to the light source to modulate the light emitted from the light source such that the blood constituent determining device, which is adapted to control the photo-detector and the frequency modulating means, can perform frequency modulation measurements for determination of the lifetime.

All references to "a/an/the [element, device, component, means, step, etc] "are to be interpreted as referring to at least one instance of the element, device, component, means, step, etc., unless explicitly stated otherwise.

Further objectives of, features of, and advantages with, the present invention will become apparent when studying the following detailed disclosure, the drawings and the appended claims. Those skilled in the art will realize that different features of the present invention can be combined to create embodiments other than those described in the following.

Brief Description of the Drawings

The above, as well as additional objects, features and advantages of the present invention, will be better understood through the following illustrative and non- limiting detailed description of preferred embodiments of the present invention, with reference to the appended drawings, in which:

Figure 1 shows an implantable sensor.

Figure 2 shows a medical lead according to an aspect of the present invention.

Figure 3 shows an implantable device according to another aspect of the present invention.

Figure 4 shows a block diagram of a method for determining a concentration of a substance and/or molecule in blood or tissue of a patient.

All the figures are schematic, not necessarily to scale, and generally only show parts which are necessary in order to elucidate the invention, wherein other parts may be omitted or merely suggested.

Description of Preferred Embodiments

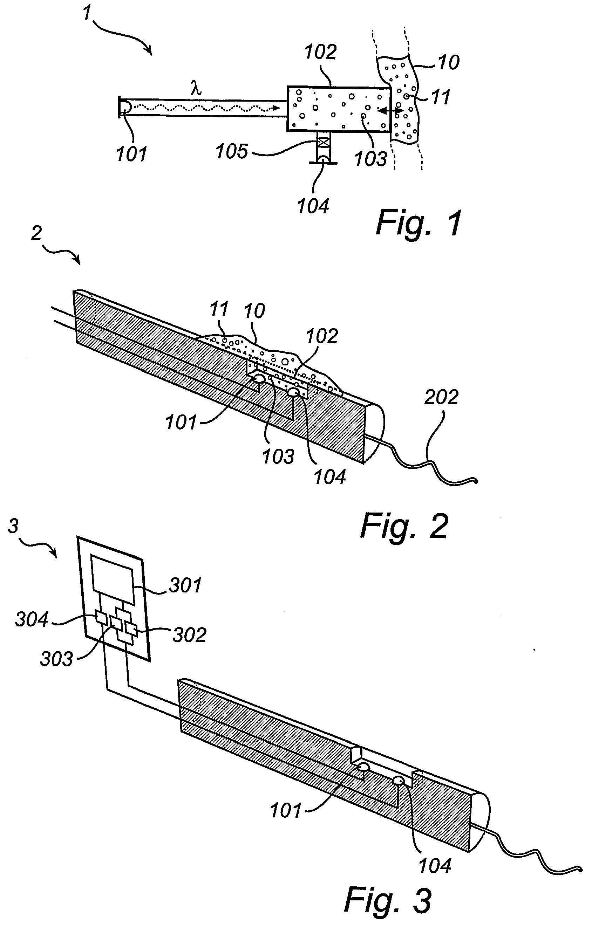

Figure 1 shows an implantable sensor according to an embodiment. The sensor 1 comprising a carrier 102 in which photoluminescent molecules 103 are embedded. The carrier 102 is partially in contact with a region 10 of a patient comprising the molecules and/or substances 11 for which the concentration shall be determined. The sensor 1 further comprises a light source 101 and a photo-detector 104 which are optically connected to the carrier 102 such that the light emitted from the light source 101 can excite the photoluminescent molecules 103 and such that the light emitted from the photoluminescent molecules 103 in response to this excitation can be detected by the photo-detector 104. The carrier 102 is selectively permeable to the molecules and/or substances 11 for which the concentration has to be determined such that these molecules 11 can diffuse from the region 10 of the patient into the carrier 102. The photoluminescent molecules 103 are selected to react with the molecules and/or substances 11 coming from the region 10 in such a manner that the characteristics of the light emitted from the photoluminescent molecules 103 is altered because of the reaction. Depending on the concentration of the molecules and/or substances 11 in the region 10 of the patient, the characteristics of the light emitted from the photoluminescent molecules 103 will then be altered to different degrees. Thus, a determination of the concentration of the molecules 11 in the region 10 of the patient is enabled.

In an embodiment, the light source 101 and the photo-detector 104 are directly arranged at a side of the carrier 102 or in the carrier 102. In another embodiment, the light source 101 and the photo-detector 104 are optically connected to the photoluminescent molecules 103 comprised in the carrier 102 by means of optical fibers. The optical fibers may be arranged between the light source 101 and the photoluminescent molecules 103 and/or between the photo-detector 104 and the photoluminescent molecules 103, which means that the light source 101 and the photo-detector 104 can be arranged at a certain distance from the analyzed region 10. The optical fibers are used to guide light from the light source and to guide light to the photo-detector, respectively. Using optical fibers to connect the light source 101 and the photo-detector 104 enables to reduce the size of the part of the sensor 1 that has to be placed in contact with the region 10 of the patient, which is advantageous if the space at the region 10 of the patient is limited.

In an embodiment, the light source 101 and the photo-detector 104 are fabricated on a same substrate using e.g. standard semiconductor technology. The carrier 102 is then made of a material, such as a silicon adhesive, like e.g. the commercially available Rehau RAU-SIK SI-1511, containing the photoluminescent molecules and spread over the substrate. The substrate can then be used as a support for the light source 101, the photo-detector 104 and the carrier 102. Such a substrate would also enable easy connection between the light source 101, the photo-detector 104 and other components used to analyze the signal output of the photo-detector 104. The thickness of the adhesive film containing the photoluminescent molecules would typically be comprised between 0.03 and 3 mm.

In yet a further embodiment, the light source 101 and the photo-detector 104 can be fabricated on two separate substrates and joined together by means of the carrier 102, e.g. the silicon adhesive mentioned above, containing the photoluminescent molecules 103.

In an embodiment, a filter 105 may be arranged in front of the photo-detector 104 to select the range of wavelength that shall be transmitted to the photo-detector. In particular, the filter 105 is used to eliminate the part of the excitation light emitted by the light source 102 that is reflected by the photoluminescent molecules 103 or by the carrier or carrier matrix 102.

In further embodiments, the carrier 102 can be made as a material comprised in the group of silicone rubber (also known as polydimethylsiloxane), organosubstitute silicones such as poly(R-R'-siloxane) wherein R and R' are one of the following {methyl, ethyl, propyl, butyl, "alkyl", "aryl", phenyl and "vinyl"} and not necessarily equal to each other, polyurethane, poly(1-trimethylsily-1-propyne), polystyrene, poly(methylmethacrylate), poly(vinyl chloride), poly(isobutylmethacrylate), poly(2,2,2-trifluoroethylmethacrylate), ethylcellulose, cellulose acetobutyrate, cellulose acetate, gas-permeable polytetrafluoroethylene and thermoplastic polyurethanes and copolymers, in which material the photoluminescent molecules 103 are embedded. Further, these materials, in particular silicone rubber, thermoplastic polyurethanes and copolymers, gas-permeable polytetrafluoroethylene, are well adapted in the present invention since they are implantable biomaterials. In a particular embodiment, silicone rubber is well adapted if the sensor 1 is used to determine the concentration of oxygen in the region 10 since its permeability to oxygen is about ten times higher than in most polymeric materials, namely $6.95 \times 10^{11}$ cm$^{-2}$s$^{-1}$Pa$^{-1}$, which corresponds to a diffusion coefficient of about $1.45 \times 10^{-5}$ cm$^2$s$^{-1}$. Thus, the diffusion of oxygen into silicone rubber will be rapid enough for measuring fast changes in the oxygenation of the region of the patient. However, other materials than silicone rubber and polyurethane may be used, such as polystyrene, the requirement being that the diffusion coefficient of the material for oxygen shall be sufficient to sense the changes in the oxygenation of the region 10. The high oxygen permeability of silicone rubber allows high flexibility on the design. Thus, a thick layer of silicone rubber could be used. In addition, the sensor 1 would remain sufficiently sensitive even if some limited amount of tissue overgrowth occurred at the side of the carrier 102 in contact with the region 10 of the patient.

The light source 101 may be a solid state light source, preferably a light emitting diode, which is advantageous since light emitting diodes are small and can therefore easily be incorporated in the sensor 1. Thus, a compact sensor can be realized, which alleviates the requirement on space close to the region 10 at which the concentration of a substance and/or molecule is to be determined. Light emitting diodes are also advantageous since the intensities, wavelengths, and time responses are controllable. Light emitting diodes can emit at various ranges, which generally extend from 390 nm to 1650 nm although not any wavelength of this range may be achieved.

Further, light emitting diodes have a rather long lifetime as compared to most common light sources and the wavelength range at which they emit can accurately be selected. In particular, the wavelength of the light source has to be selected with respect to the photoluminescent molecules employed for the detection. In general, the absorption range for most photoluminescent molecules extends from 350 to 800 nm. However, each particular photoluminescent molecule has its own absorption band and the appropriate wavelength of the light source depends accordingly. As an example, oxyphors have an absorption maximum near 636 nm, which corresponds to the wavelength required to excite the photoluminescent molecules. Thus, a light source 101 emitting at this wavelength is needed. Such a light source can be commercially available light emitting diodes, like the type Toshiba LED lamp TLSU1102 which has an emission peak at 20 mA of 636 nm with intensity of 200 Mcd. A person skilled in the art would understand that other light emitting diodes or light sources capable of emitting light in the range of 610-655 nm would be suitable to excite oxyphors.

The sensitivity of the photo-detector 104 of the implantable sensor 1 depends on the wavelength range at which the photoluminescent molecules 103 emit. Generally, the range of wavelength at which photoluminescent molecules emit extend from 350 to 1800 nm, in particular in the range of 350-800 nm. Thus, the photo-detector 104 shall be sensitive in this range of wavelength. As the wavelength at which a selected photoluminescent molecules emit is known, the sensitivity of the photo-detector can be selected accordingly. As an example, oxyphors emit light at about 800 nm, which corresponds to near infrared. In this case, the photo-detector may be a commercially available planar InGaAs photodiode, which is sensitive to red and infrared light, i.e. in the spectral range of 800-1800 nm. Such a detector has a response time of up to 120 MHz, which is sufficiently rapid for measuring photoluminescent lifetimes and sufficiently quantitative for steady state measurements of intensities as well. The measurements of lifetimes and intensities will be described in more details later. In particular, the photo-detector may be one of the group comprised of a pn photodiode, a pin photodiode, a Schottky photodiode, an avalanche photodiode, a silicon photodiode, a planar InGaAs photodiode, a SiGe-based optoelectronic circuit and a InGaN/GaN multiple quantum well pn junction.

The photoluminescent molecules 103 should be selected in accordance with the substances and/or molecules for which the concentration has to be determined. The sensor 1 may be adapted to detect several kinds of substances and/or molecules comprised in the region 10 at the condition that these molecules can diffuse through the carrier 102 and react with photoluminescent molecules such that the characteristics of the light emitted by the photoluminescent molecules is altered. In particular, the sensor 1 may be adapted to determine the concentration of oxygen and/or the concentration of carbon dioxide in the region 10 of a patient. In another embodiment, the sensor 1 may be adapted to determine the concentration of molecules representative of the PH-value of the region 10, thereby a sensor for determining the PH-value of a region 10 can be achieved. Further, the molecule and/or substance for which the concentration is to be determined may be one of the group comprised of glucose, urea, ammonia, lactose, hormones and insulin. A list of photoluminescent molecules with which glucose concentrations and PH-values may be determined is given later.

The molecules and/or substances for which the concentration is to be determined preferably are gas molecules since the diffusion of gas molecules through the carrier is facilitated as compared to other types of molecules. However, the sensor would also function with liquid substances and/or molecules.

The photoluminescent molecules may be fluorescent molecules or phosphorescent molecules. Although it would be preferable to use fluorescent molecules as they are generally more stable over time, it is preferable to use phosphorescent molecules as phosphorescence leads to longer timescale, which then facilitates the design of the electronics in the sensor.

One of the requirements on the photoluminescent molecules is that they shall react with the molecules and/or substances for which the concentration has to be determined in such a manner that the characteristics of the light that they emit is altered in accordance with the concentration of the molecules and/or substances to be determined. The photoluminescent molecules preferably have a long longetivity of luminescence, high quantum yield, good chemical stability in implanted environment and high selectivity to the molecules and/or substances for which the concentration is to be determined. The photoluminescent molecules shall be stable enough such that the intensity of the light that they emit remains sufficient over a long period of time, e.g. ten years. Further, if the sensor is designed to determine the concentration of oxygen in blood, it is preferable that the photoluminescent molecules are not sensitive to other gases than oxygen such as carbon monooxide which can also be found in blood. The photoluminescent molecules shall also be easily bounded to the material of the carrier, i.e. they should be compatible with the characteristics of the carrier.

The photoluminescent molecules 103 may e.g. be immobilized by means of covalent bonding with the material of the carrier 102. This can be done by a wide number of possible chemical synthetic pathways. In an embodiment, the photoluminescent molecules are bounded to the carrier by physisorption or chemisorption to an immobile filler comprised in the carrier, which is especially useful for silicone rubber which normally contains high surface area active silica as a reinforcing filler. In another embodiment, if the photoluminescent molecules are ions, the molecules can be ionically bonded to the carrier. In another embodiment, the photoluminescent molecules are covalently bonded to the carrier, especially as a pendant substitution group to the polymer used as a carrier. In yet a further embodiment, the photoluminescent molecules are immobilized by size immobilization which prevents large molecules from diffusing in the carrier.

In the case of pH, glucose (monosaccharides) and $CO_2$ sensitive photoluminescent molecules, a hydrophilic substrate coated by the carrier in which the photoluminescent molecules are embedded may be used. Alternatively, a hydrophilic material could be used for the carrier.

Pyrene and various pyrene derivatives are e.g. well suitable for a carrier made of silicone rubber.

In another embodiment, two different types of photoluminescent molecules may be embedded in the carrier such that concentrations of two different types of molecules and/or substances may be determined. The two different types of photoluminescent molecules would then preferably emit at two different wavelengths and a first analysis of the wavelength spectrum, based on the signals output by the photo-detector, would indicate which of the signals refer to which type of photoluminescent molecules.

Photoluminescent molecules that may be used to determined the concentration of oxygen are molecules comprised within the group of pyrene, pyrene derivatives (vinylpirene, methoxypyrene), a polyaromatic carrier, an ionic probe (ruthenium trisbypyridine) and Pd-tetra (4-carboxyphenyl) benzoporphin (Oxyphor). Additional. examples are naphthalene derivatives (e.g. 2-dimethylamino-6-lauroylnaphthalene); polypyridyl complexes of transition metals, particularly Ruthenium, Osmium, or Rhenium containing fluorescent molecules {e.g. Ru(bipy)(3)(2+) (tris(2,2'-bipyridyl)ruthenium(II) chloride hexahydrate) and Ru(phen)(3)(2+) (tris(1,10-phenanthroline)ruthenium(II) chloride hydrate)}; fullerenes (C60 and C70); decacyclene; perylene and perylene derivatives (e.g. - perylene dibutylate); and metalloporphines especially Pt(II), Zn(II) and Pd(II) variants.

Photoluminescent molecules that may be used to determined the concentration of glucose are boronic acid containing fluorophores (e.g. 4'-dimethylaminostilbene-4-boronic acid; 4'-cyanostilbene-4-boronic acid; 4-amino-3-fluorophenylboronic acid; and 4-carboxy-3-fluorophenylboronic acid).

Photoluminescent molecules that may be used to determined the PH-value of the region are pyrene derivatives (e.g hydroxypyrene sulfonic acid; aminopyrene; N-(3-Pyrene)succinimidothioethanol); β-methylumbelliferone; fluorescien derivatives (e.g. Carboxynaphthofluorescein; 5,6-carboxyfluorescein; fluoresceinamine; bis-carboxyethylcarboxyfluorescein) cynanine derivatives (e.g. benzothiazolium trimethine cyanine), seminaphthorhodafluor (SNARF) and carboxyseminaphthofluorescein (SNAFL) derivatives.

In further embodiments, the concentration of the photoluminescent molecules 103 is comprised in the range of 20 ppb to 2% w/w, preferentially in the range of 0.1 - 500 ppm, and more preferentially at approximately 5 ppm.

Generally, the principle of the sensor 1 is based on the deactivation or quenching of the luminescence of the photoluminescent molecules by the molecules and/or substances for which the concentration has to be determined, called quenchers in the following. The quenchers, e.g. oxygen molecules, are capable of deactivating the luminescence of the photoluminescent molecules, i.e. that excited photoluminescent molecules do not give off light because of the presence of and the reaction with quenchers. The effect of the quenching is proportional to the amount of quenchers. The deactivation process is conventionally modeled by the Stern-Volmer relationship, which is expressed as follows:

$$I(t) = I(0) \times \exp(-k_{obs} \times t) \qquad (1)$$

where I(t) is the transient intensity of the luminescence at time t, I(0) is the intensity of the luminescence at time t = 0, t is the time and $k_{obs}$ is the empirically observed pseudo-first order rate constant characteristic of the decay rate of the luminescence of the photoluminescent molecules in presence of quenchers.

The coefficient $k_{obs}$ can be expressed as a function of the first order decay rate constant $k_1$ representative of

the decay rate of the luminescence of the photoluminescent molecules without the presence of quenchers, the second order rate constant of quenching kq representative of the rate at which the photoluminescent molecules are quenched by the quenchers and [Q] the concentration of quenchers, as follows:

$$k_{obs} = k_q \times [Q] + k_1 \qquad (2)$$

By integration over time of the Stern-Volmer relationship (1) and using equation (2), the concentration of quenchers can be expressed as:

$$[Q] = \frac{k_1}{k_q} \times \left( \frac{I_0}{I_f} - 1 \right) \qquad (3)$$

where If is the steady state intensity of luminescence. Equation 3 shows that there is a relationship between the steady state intensity and the concentration of the quenchers, from which relationship the concentration of the molecules and/or substances can be determined. Thus, the measurement of the intensity of the light emitted by the photoluminescent molecules results in the concentration of the molecules and/or substances 11 in the region 10 of the patient.

Starting from equations (1) and (2), the transient intensity of the luminescence can also be expressed as:

$$\ln\left( \frac{I(0)}{I(t)} \right) = \left( k_q \times [Q] + k_1 \right) \times t \qquad (4)$$

Thus, time-resolved measurement showing the time-resolved decay of the luminescence as a function of time enables also to determine the concentration of quenchers. In practice, the natural logarithm of intensity is plotted as a function of time and the concentration of quenchers is extracted from the slope of the resulting straight line. Time-resolved measurements are also called lifetime measurements since this type of measurements is a way of measuring the lifetime of the luminescence, i.e. the time it takes for the luminescence to decay to a minimal value (in principle to a "zero" value). In other words, the lifetime represents the time it takes for extinction of the luminescence of the photoluminescent molecules. Such measurements are advantageous since they are not dependent on the amount of photoluminescent molecules at the time of the measurement. Two different alternatives for measuring lifetime will be described later.

The signals output by the photo-detector may also be analysed to determine the wavelength of the light emitted by the photoluminescent molecules. When the sensor is employed to determine the concentration of a substance such as oxygen in blood, the wavelength of the light emitted by the photoluminescent molecules will generally be the same and not depend on the amount of oxygen molecules in blood. However, the sensor may be used to determine the PH-value of the region 10. In this case, the photoluminescent molecules are chosen in such a manner that they react with e.g. $H_3O^+$ and $OH^-$ molecules of the region. As an example, aminopyrene can be used as a photoluminescent molecule. The amino-group of aminopyrene molecules has the ability to react with hydrogen such that it becomes negatively charged if the PH-value is negative and positively charged if the PH-value is positive. As the wavelength at which aminopyrene molecules emit depends on whether they are positively or negatively charged, i.e. depends on the PH-value, the analysis of the wavelength of the light enables to determine the PH-value of the region 10 of the patient.

The carrier 102 is, at least partially, in contact with a region 10 of the patient where the concentration of the molecule and/or substance has to be determined. This region may in principle be any region but in particular, the sensor may be placed at the heart of the patient, in arterial blood, in venous blood or implanted at another organ of the patient. Arranged at such regions, it is probable that the sensor may physically be subjected to little tissue overgrowth. However, the sensor of the present invention would still function since the optical measurement occurs inside the carrier 102 and not directly in the blood or the tissue. In other words, the light emitted from the light source and the light emitted from the photoluminescent molecules do not have to pass through an interface in contact with the region of the patient, which would, otherwise, enhance tissue overgrowth.

In an embodiment, the light source 101 and the photo-detector 104 are hermetically isolated from the region 10 of the patient at which the carrier 102 is arranged.

In the following, some examples of sensors are given for determining the concentration of oxygen, glucose and carbon dioxide in blood. First, three alternatives are presented to implement a sensor for determining the concentration of oxygen in blood.

In a first alternative, the carrier of the sensor is made of poly(dimethylsiloxane), PDMS in short. This material presents the advantage of being flexible, and has suitable biocompatibility and biostability characteristics. In addition, this material has the advantage of being highly permeable to oxygen, which imparts high sensitivity to the oxygen sensor. Decacyclene (DCY in the following), with a concentration in the order of 5 ppm in the carrier, is a suitable probe molecule (or photoluminescent molecule) for sensing the concentration of oxygen since oxygen molecules can quench the fluorescence of DCY very efficiently. However, DCY needs to be solubilized in PDMS, which can e.g. be implemented by introducing tertiary butyl groups to increase solubility. Further, inclusion of a vinyl group enables DCY to be immobilized in the carrier (PDMS) by addition reaction to pendant vinyl groups on a partially vinyl-substituted component of the PDMS precursor. Alternatively, alkyloxy-groups can be included on DCY to enable a condensation reaction that immobilizes DCY with the PDMS. Further, as DCY has an excitation maximum near 380 nm, an inexpensive light source such as a LED emitting in the blue or near UV portion of the spectrum can be used. The light source may be a commercially available blue light laser with an emission maximum at 405 nm. As DCY emits fluorescent light with maximum intensity near 510 nm, a suitable photo-detector is a photodiode which is sensitive to this wavelength and which has a high speed response suitable for lifetime measurements. A possible choice is a silicon-based avalanche photodiode, which provides very high sensitivity in the range of 450-1000 nm and which has a maximum sensitivity in the range of 600-800 nm. Further, a silicon-based avalanche photodiode provides the advantage of being very little sensitive to the light emitted from the blue LED light source, thereby reducing the need for optical filtering to shield the detector from scattered light from the light source.

In a second alternative, the carrier of the sensor may be made of polystyrene (PS), which presents good mechanical properties. Although, the permeability of oxygen in PS is lower than the permeability of oxygen in PDMS, PS presents the advantage that the probe molecules dissolved in PS do not easily diffuse, which means that chemical modifications to immobilize the probe molecules are usually unnecessary. On the other hand, a thin protective coating of PDMS might be useful to protect PS from degradation by biological processes, thereby ensuring a long lifetime to the implanted sensor. In this embodiment, perylene dibutyrate may be used as probe molecules. As these photoluminescent molecules have an excitation maximum near 457 nm and an emission maximum near 512 nm, inexpensive components for the light source, e.g. a blue LED with a light intensity maximum at 460nm such as the commercially available Opto Technology OTL460A-1-1-46-D-2, and for the photo-detector, such as a silicon-based avalanche photodiode, may be used. Further, perylene dibutyrate has a high degree of photostability as compared to other probe molecule types. The photoluminescent molecules may have a concentration of about 0.1% w/w in the carrier.

In a third alternative, the sensor may be made of Pd(II) octaethylporphyrin-ketone (PdOEPK) dispersed with a concentration in the range of 0.5% w/w (500ppm) in a PS carrier. Such a sensor presents good photostability, and the photoluminescent molecules have a long lifetime, which facilitates oxygen quantification by determination of luminescent lifetime changes. The sensor is therefore particularly suitable for determining physiological oxygen concentrations. Further, as PdOEPK has excitation maxima around 410 nm and 603 nm and an emission maximum around 790 nm, an inexpensive and highly efficient red LED may be used as a light source and a near infrared detector such as an InGaAS avalanche photo-diode may be used as a photo-detector. The optics of the sensor is also simplified as PdOEPK presents a high quantum yield of luminescence at body temperatures.

Then, an alternative to implement a sensor for determining the concentration of glucose in blood is presented. In this embodiment, the carrier may be made of a hydrophilic polymer to facilitate transport of glucose to the probe molecules immobilized in the carrier or in the carrier film. Films based on polyvinyl alcohol are preferred as they present good biocompatibility and flexibility. Such films have the ability to contain boronic acid-containing fluorophores, such as 4'-dimethylaminostilbene-4-boronic acid (DSTBA), which may be used as photoluminescent molecules in this embodiment. The concentration of the photoluminescent molecules in the carrier may be in the order of 50 ppm. These photoluminescent molecules have an excitation maximum near 340 nm and an emission maximum between 450 and 500 nm depending on the concentration of glucose or other monosaccharides. Thus, a suitable excitation source may be a UV LED with a peak of emission at around 370 nm and a suitable detector may be a silicon-based avalanche photodiode.

Then, an alternative to implement a sensor for determining the concentration of carbon dioxide in blood is presented. In this embodiment, PDMS may be selected as material for the carrier to immobilize the probe molecules since PDMS permeable to carbon dioxide but impermeable to ions, thus minimizing interference by e.g. Cl ions and/or pH effects. PDMS is biostable and biocompatible, thus suitable for long term implant. 1-hydroxypyrene-3,6,8-trisulfonate (HPTS), HPTS in the following, may be used as photoluminesecent molecules since its fluorescence characteristics are correlated to the concentration of carbon dioxide. HTPS has an excitation maximum near 470 nm and may therefore be excited by a blue LED having a light intensity maximum at 460 nm, such as the commercially available "Opto Technology OTL460A-1-1-46-D-2". HTPS has an emission maximum near 510nm, which may be detected by a silicon-based avalanche photodiode. Further, HTPS presents the advantage of being very little affected by oxygen or other gases found in significant concentrations in a physiological environment. The concentration of HTPS in the carrier may be in the order of 500 ppm.

With reference to figure 2, an aspect of the present invention is presented.

Figure 2 shows a medical lead according to an embodiment of the present invention. The medical lead typically comprises an implantable sensor 1 such as the sensor described above. The medical lead 2 shown in figure 2 is an elongated lead in which a depression is formed. The lead would preferably be cylindrical with a circular or elliptic section but the lead may also have other types of sections. The depression extends only on a short portion of the lead 2. Photoluminescent molecules 103 embedded in the carrier 102 are placed inside the depression. In the embodiment shown in figure 2, the light source 101 and the photo-detector 104 are also arranged inside the depression. However, as mentioned above for the implantable sensor 1 described with reference to figure 1, the light source 101 and the photo-detector 104 may be arranged at a distance from the carrier and optically connected to the carrier by means of optical fibers. The configuration shown in figure 2 is particularly well adapted for determining the concentration of a substance and/or molecule comprised in the blood or in the tissue of a patient. The medical lead could be arranged in an artery or a vein but would preferably be arranged at the heart of a patient where there is a high blood flow. The medical lead therefore comprises a screw 202 used to attach the medical lead to the heart.

With reference to figure 3, another aspect of the present invention is presented.

Figure 3 shows an implantable device 3 such as a pacemaker implantable cardioverter-defibrillator (ICD) or similar cardiac simulation device according to an embodiment of the present invention. The implantable device 3 is connected to a medical lead similar to that described with reference to figure 2, i.e. a medical lead comprising an implantable sensor similar to that described with reference to figure 1. Only some of the features of the sensor are represented in figure 3. However, a sensor such as any one of the sensors described in accordance with the embodiments cited above could be used. The implantable device shown in figure 3 further comprises a blood constituent determining device 301 adapted to determine the concentration of the quenchers based on the signals output from the photo-detector 104. The determination may be based on the above described equations 3 and 4.

The implantable device 3 may also comprise a pulse-generating means connected to the light source 101 and the constituent determining device 301 such that light pulses may be generated to perform time-resolved measurements, such as those explained with reference to equation 4. For example, a 4 $\mu$s light pulse may be generated to excite the photoluminescent molecules 103 arranged in the carrier 102. Measurements of the intensity at different time points like 30, 50, 120, 180, 240, 420 and 2500 $\mu$s after the generation of the light pulse may be performed to calculate the characteristic lifetime of the photoluminescent molecules 103 and to determine the concentration of the quenchers in the region 10 in accordance with equation (4).

The implantable device may also comprise a frequency modulating means 303 connected to the light source 101 and the constituent determining device 301 such that an array of harmonics (with for instance 100-200 frequencies) is used to modulate the light source 101. Using such a technique, lifetimes in the range of 10-3000 $\mu$s may be measured.

The implantable device may also comprise an amplifier 304 adapted to amplify the signals output from the photo- detector 104.

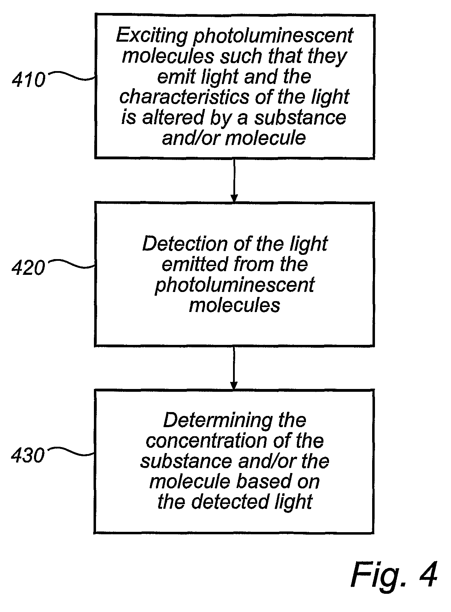

Figure 4 shows a block diagram of a method for determining a concentration of a substance and/or molecule in blood or tissue of a patient according to an embodiment of the present invention. In this method, a first step (step 410) consists of, during measurements sessions, exciting photoluminescent molecules embedded in a carrier selectively permeable to the substance and/or molecule such that the photoluminescent molecules emit light. In a second step, step 420, the light emitted from the photoluminescent molecules is detected and in a third step, step 430, the concentration of the substance and/or the molecule is determined based on characteristics of the detected light.

The present invention is applicable in implantable devices for determining a concentration of a substance and/or molecule comprised in the blood composition or in the tissue of a patient. Typically, the present invention is applicable to determine the concentration of oxygen in blood or the saturation of oxygen in blood for patient having a pacemaker.

Although the invention above has been described in connection with preferred embodiments of the invention, it will be evident for a person skilled in the art that several modifications are conceivable without departing from the scope of the invention as defined by the following claims.

## Claims

1. A medical lead (2) comprising a sensor (1) for determining a concentration of a substance and/or a molecule (10) in blood or tissue of a patient, wherein said sensor (1) comprises at least one type of photoluminescent molecules (103) embedded in a carrier (102) selectively permeable to said substance and/or molecule (10), wherein a part of said carrier is in contact with a region of said patient, which region (10) comprises said substance and/or molecule (11), and wherein at least one light source (101), adapted to, during measurement sessions, emit light at least at a first wavelength, and at least one photo-detector (104) are optically connected to said carrier (102), wherein said photoluminescent molecules (103) emit light in response to excitation by said light emitted from said at least one light source (101) and wherein said substance and/or molecule (11) reacts with said photoluminescent molecules (103) to alter characteristics of said light emitted from said photoluminescent molecules (103), wherein said at least one photo-detector (104) is adapted to detect said light emitted from said photoluminescent molecules (103) and to provide output signals representative of said characteristics to determine said concentration of a substance and/or a molecule (11), said medical lead (2) in the form of an elongated cylindrical lead with a circular or elliptic cross-section, **characterized in that**

said sensor (1), in said medical lead (2), comprises said at least one light source (101) and said at least one photo-detector (104);

said medical lead (2) comprises a depression in its lateral surface, said depression extending along a portion of said medical lead (2); and

said photoluminescent molecules (103) embedded in said carrier (102) are placed inside said depression in said lateral surface of said medical lead (2).

2. The medical lead as defined in claim 2, wherein said light source (101) and said photo-detector (104) are arranged inside said depression.

3. The medical lead as defined in claim 1 or 2, wherein said photoluminescent molecules (103) are adapted to react with a substance and/or a molecule (11) representative of the pH-value of said region (10).

4. The medical lead as defined in any one of claims 1-3, wherein said substance and/or molecule (11) is one of the group comprised of oxygen, carbon dioxide, glucose, urea, ammonia, lactose, hormones and insulin for determining the concentration of said substance and/or molecule in said region.

5. The medical lead as defined in any one of claims 1-4, wherein said photoluminescent molecules (103) are phosphorescent molecules.

6. The medical lead as defined in any one of claims 1-5, wherein said sensor (1) is placed at the heart of said patient, in arterial blood, in venous blood, or in the tissue of a blood-containing organ of said patient.

7. The medical lead as defined in any one of claims 1-6, wherein said at least one light source (101) and said at least one photo-detector (104) are hermetically isolated from said region (10).

8. The medical lead as defined in any one of claims 1-7, wherein said characteristics is at least one of the intensity of said light emitted from said photoluminescent molecules (103), the lifetime of said light emitted from said photoluminescent molecules (103) and the wavelength of said light emitted from said photoluminescent molecules (103).

9. The medical lead as defined in any of the claims 1-8, wherein said part of said carrier (102) is in direct contact with said region (10) of said patient.

10. An implantable device (3) connected to a medical lead as defined in any of claims 1-8 and comprising:

    a blood constituent determining device (301) adapted to receive output signals from said photo-detector (104) to determine the concentration of said substance and/or molecule (11) based on said received signals.

11. The implantable device as defined in claim 10, wherein said characteristics is at least the lifetime of said light emitted from said photoluminescent molecules (103) and wherein said blood constituent determining device (301) is adapted to determine the concentration of said substance and/or molecule (11) based on said lifetime.

12. The implantable device as defined in claim 11, further comprising a pulse generating means (302) connected to said at least one light source (101) to cause the light source (101) to emit light pulses, wherein said blood constituent determining device (301) is adapted to control said at least one photo-detector (104) and said pulse generating means (302) to perform time resolved measurements for determination of said lifetime.

13. The implantable device as defined in claim 11, further comprising a frequency modulating means (303) connected to said at least one light source (101) to modulate the light emitted from said at least one light source (101), wherein said blood constituent determining device (301) is adapted to control said at least one photo-detector (104) and said frequency modulating means (303) to perform frequency modulation measurements for determination of said lifetime.

14. The implantable device as defined in any one of claims 10-13, further comprising an amplifier (304) for amplifying said output signals sent from said at least one photo-detector (104) to said blood constituent determining device (301).

**15.** The implantable device as defined in any of claims 10-14, wherein an optical filter (105) is arranged at said photo-detector (104) to selectively transmit said light emitted by said photoluminescent molecules (103) to said at least one photo-detector (104).

**Patentansprüche**

**1.** Medizinische Leitung (2), umfassend einen Sensor (1) zum Bestimmen einer Konzentration einer Substanz und/oder eines Moleküls (10) im Blut oder Gewebe eines Patienten, wobei der Sensor (1) mindestens eine Art von fotolumineszierenden Molekülen (103) umfasst, die in einen für die Substanz und/oder das Molekül (10) selektiv durchlässigen Träger (102) eingebettet sind, wobei ein Teil des Trägers mit einer Region des Patienten in Kontakt ist, wobei die Region (10) die Substanz und/oder das Molekül (11) umfasst, und wobei mindestens eine Lichtquelle (101), die während Messreihen zum Emittieren mindestens einer ersten Wellenlänge angepasst ist, und mindestens ein Fotodetektor (104) mit dem Träger (102) optisch verbunden sind, wobei die fotolumineszierenden Moleküle (103) als Antwort auf Anregung durch das von der mindestens einen Lichtquelle (101) emittierte Licht Licht emittieren und wobei die Substanz und/oder das Molekül (11) mit den fotolumineszierenden Molekülen (103) reagieren, um Eigenschaften des von den fotolumineszierenden Molekülen (103) emittierten Lichts zu verändern, wobei der mindestens eine Fotodetektor (104) dazu angepasst ist, das von den fotolumineszierenden Molekülen (103) emittierte Licht zu erfassen und für die Eigenschaften repräsentative Ausgabesignale bereitzustellen, um die Konzentration einer Substanz und/oder eines Moleküls (11) zu bestimmen, wobei die medizinische Leitung (2) die Form einer länglichen zylinderförmigen Leitung mit einem kreisförmigen oder ellipsenförmigen Querschnitt aufweist, **dadurch gekennzeichnet, dass**
der Sensor (1) in der medizinischen Leitung (2) die mindestens eine Lichtquelle (101) und den mindestens einen Fotodetektor (104) umfasst;
die medizinische Leitung (2) in ihrer Seitenfläche eine Vertiefung umfasst, wobei sich die Vertiefung entlang eines Abschnitts der medizinischen Leitung (2) erstreckt; und
die in den Träger (102) eingebetteten fotolumineszierenden Moleküle (103) innerhalb der Vertiefung in der Seitenfläche der medizinischen Leitung (2) angeordnet sind.

**2.** Medizinische Leitung nach Anspruch 2, wobei die Lichtquelle (101) und der Fotodetektor (104) innerhalb der Vertiefung angeordnet sind.

**3.** Medizinische Leitung nach Anspruch 1 oder 2, wobei die fotolumineszierenden Moleküle (103) dazu angepasst sind, mit einer Substanz und/oder einem Molekül (11) zu reagieren, die für den pH-Wert der Region (10) repräsentativ sind.

**4.** Medizinische Leitung nach einem der Ansprüche 1 bis 3, wobei die Substanz und/oder das Molekül (11) eine/s aus der Gruppe umfassend Sauerstoff, Kohlendioxid, Glukose, Harnstoff, Ammoniak, Laktose, Hormone und Insulin zum Bestimmen der Konzentration der Substanz und/oder des Moleküls in der Region ist.

**5.** Medizinische Leitung nach einem der Ansprüche 1 - 4, wobei die fotolumineszierenden Moleküle (103) phosphoreszierende Moleküle sind.

**6.** Medizinische Leitung nach einem der Ansprüche 1 - 5, wobei der Sensor (1) am Herzen des Patienten, in arteriellem Blut, in venösem Blut oder im Gewebe eines bluthaltigen Organs des Patienten angeordnet wird.

**7.** Medizinische Leitung nach einem der Ansprüche 1 - 6, wobei die mindestens eine Lichtquelle (101) und der mindestens eine Fotodetektor (104) von der Region (10) hermetisch isoliert sind.

**8.** Medizinische Leitung nach einem der Ansprüche 1 bis 7, wobei es sich bei den Eigenschaften um mindestens eine aus der Intensität des von den fotolumineszierenden Molekülen (103) emittierten Lichts, der Lebensdauer des von den fotolumineszierenden Molekülen (103) emittierten Lichts und der Wellenlänge des von den fotolumineszierenden Molekülen (103) emittierten Lichts handelt.

**9.** Medizinische Leitung nach einem der Ansprüche 1 - 8, wobei der Teil des Trägers (102) in direktem Kontakt mit der Region (10) des Patienten ist.

**10.** Implantierbare Vorrichtung (3), die mit einer medizinischen Leitung nach einem der Ansprüche 1 - 8 verbunden ist und Folgendes umfasst:

> eine Vorrichtung (301) zum Bestimmen von Blutbestandteilen, die dazu angepasst ist, Ausgabesignale vom Fotodetektor (104) zu empfangen, um die Konzentration der Substanz und/oder des Moleküls (11) auf der Basis der empfangenen Signale zu bestimmen.

11. Implantierbare Vorrichtung nach Anspruch 10, wobei es sich bei den Eigenschaften um mindestens die Lebensdauer des von den fotolumineszierenden Molekülen (103) emittierten Lichts handelt und wobei die Vorrichtung (301) zum Bestimmen von Blutbestandteilen dazu angepasst ist, die Konzentration der Substanz und/oder des Moleküls (11) auf der Basis der Lebensdauer zu bestimmen.

12. Implantierbare Vorrichtung nach Anspruch 11, ferner umfassend einen mit der mindestens einen Lichtquelle (101) verbundenen Impulserzeuger (302) zum Bewirken dessen, dass die Lichtquelle (101) Lichtimpulse emittiert, wobei die Vorrichtung (301) zum Bestimmen von Blutbestandteilen dazu angepasst ist, den mindestens einen Fotodetektor (104) und den Impulserzeuger (302) derart zu steuern, dass zur Bestimmung der Lebensdauer zeitaufgelöste Messungen durchgeführt werden.

13. Implantierbare Vorrichtung nach Anspruch 11, ferner umfassend einen mit der mindestens einen Lichtquelle (101) verbundenen Frequenzmodulator (303) zum Modulieren des von der mindestens einen Lichtquelle (101) emittierten Lichts, wobei die Vorrichtung (301) zum Bestimmen von Blutbestandteilen dazu angepasst ist, den mindestens einen Fotodetektor (104) und den Frequenzmodulator (303) derart zu steuern, dass zur Bestimmung der Lebensdauer Frequenzmodulationsmessungen durchgeführt werden.

14. Implantierbare Vorrichtung nach einem der Ansprüche 10 - 13, ferner umfassend einen Verstärker (304) zum Verstärken der von dem mindestens einen Fotodetektor (104) an die Vorrichtung (301) zum Bestimmen von Blutbestandteilen gesendeten Ausgabesignale.

15. Implantierbare Vorrichtung nach einem der Ansprüche 10 - 14, wobei am Fotodetektor (104) ein optischer Filter (105) angeordnet ist, um das durch die fotolumineszierenden Moleküle (103) an den mindestens einen Fotodetektor (104) emittierte Licht selektiv zu übertragen.

**Revendications**

1. Dérivation médicale (2) comprenant un détecteur (1) permettant de déterminer une concentration d'une substance et/ou d'une molécule (10) dans le sang ou dans un tissu d'un patient, dans laquelle ledit détecteur (1) comprend au moins un type de molécules photoluminescentes (103) incorporé dans un support (102) sélectivement perméable à ladite substance et/ou molécule (10), dans laquelle une partie dudit support est en contact avec une région dudit patient, laquelle région (10) comprend ladite substance et/ou molécule (11), et dans laquelle au moins une source de lumière (101), adaptée pour, durant des sessions de mesure, émettre de la lumière au moins à une première longueur d'onde et au moins un photodétecteur (104) sont optiquement connectés audit support (102), dans laquelle lesdites molécules photoluminescentes (103) émettent de la lumière en réponse à une excitation par ladite lumière émise depuis ladite au moins une source de lumière (101) et dans laquelle ladite substance et/ou molécule (11) réagit avec lesdites molécules photoluminescentes (103) pour altérer des caractéristiques de ladite lumière émise depuis lesdites molécules photoluminescentes (103), dans laquelle ledit au moins un photodétecteur (104) est adapté pour détecter ladite lumière émise depuis lesdites molécules photoluminescentes (103) et pour fournir des signaux de sortie représentatifs desdites caractéristiques afin de déterminer ladite concentration d'une substance et/ou d'une molécule (11), ladite dérivation médicale (2), sous la forme d'une dérivation cylindrique allongée avec une section transversale circulaire ou elliptique, étant **caractérisée en ce que** ledit détecteur (1), dans ladite dérivation médicale (2), comprend ladite au moins une source de lumière (101) et ledit au moins un photodétecteur (104) ; ladite dérivation médicale (2) comprend une dépression dans sa surface latérale, ladite dépression s'étendant le long d'une partie de ladite dérivation médicale (2) ; et lesdites molécules photoluminescentes (103) incorporées dans ledit support (102) sont placées à l'intérieur de ladite dépression dans ladite surface latérale de ladite dérivation médicale (2).

2. Dérivation médicale selon la revendication 2, dans laquelle ladite source de lumière (101) et ledit photodétecteur (104) sont agencés à l'intérieur de ladite dépression.

3. Dérivation médicale selon la revendication 1 ou 2, dans laquelle lesdites molécules photoluminescentes (103) sont adaptées pour réagir avec une substance et/ou une molécule (11) représentative de la valeur du pH de ladite région (10).

4. Dérivation médicale selon l'une quelconque des revendications 1 à 3, dans laquelle ladite substance et/ou molécule (11) est une substance ou molécule du groupe comprenant l'oxygène, le dioxyde de carbone, le glucose, l'urée, l'ammoniac, le lactose, des hormones et l'insuline pour déterminer la concentration de ladite substance et/ou molécule dans ladite région.

5. Dérivation médicale selon l'une quelconque des revendications 1 à 4, dans laquelle lesdites molécules photoluminescentes (103) sont des molécules phosphorescentes.

6. Dérivation médicale selon l'une quelconque des revendications 1 à 5, dans laquelle ledit détecteur (1) est placé au niveau du coeur dudit patient, dans le sang artériel, dans le sang veineux ou dans le tissu d'un organe dudit patient contenant du sang.

7. Dérivation médicale selon l'une quelconque des revendications 1 à 6, dans laquelle ladite au moins une source de lumière (101) et ledit au moins un photodétecteur (104) sont hermétiquement isolés de ladite région (10).

8. Dérivation médicale selon l'une quelconque des revendications 1 à 7, dans laquelle lesdites caractéristiques sont au moins une caractéristique parmi l'intensité de ladite lumière émise depuis lesdites molécules photoluminescentes (103), la durée de vie de ladite lumière émise depuis lesdites molécules photoluminescentes (103) et la longueur d'onde de ladite lumière émise par lesdites molécules photoluminescentes (103).

9. Dérivation médicale selon l'une quelconque des revendications 1 à 8, dans laquelle ladite partie dudit support (102) est en contact direct avec ladite région (10) dudit patient.

10. Dispositif implantable (3) connecté à une dérivation médicale selon l'une quelconque des revendications 1 à 8 et comprenant :

   un dispositif de détermination de constituants sanguins (301) adapté pour recevoir des signaux de sortie provenant dudit photodétecteur (104) afin de déterminer la concentration de ladite substance et/ou molécule (11) sur la base desdits signaux reçus.

11. Dispositif implantable selon la revendication 10, dans lequel lesdites caractéristiques sont au moins la durée de vie de ladite lumière émise par lesdites molécules photoluminescentes (103) et dans lequel ledit dispositif de détermination de constituants sanguins (301) est adapté pour déterminer la concentration de ladite substance et/ou molécule (11) sur la base de ladite durée de vie.

12. Dispositif implantable selon la revendication 11, comprenant en outre un moyen de génération d'impulsions (302) connecté à ladite au moins une source de lumière (101) pour amener la source de lumière (101) à émettre des impulsions de lumière, dans lequel ledit dispositif de détermination de constituants sanguins (301) est adapté pour commander ledit au moins un photodétecteur (104) et ledit moyen de génération d'impulsions (302) pour effectuer des mesures de résolution temporelle pour la détermination de ladite durée de vie.

13. Dispositif implantable selon la revendication 11, comprenant en outre un moyen de modulation de fréquence (303) connecté à ladite au moins une source de lumière (101) pour moduler la lumière émise depuis ladite au moins une source de lumière (101), dans lequel ledit dispositif de détermination de constituants sanguins (301) est adapté pour commander ledit au moins un photodétecteur (104) et ledit moyen de modulation de fréquence (303) pour effectuer des mesures de modulation de fréquence pour la détermination de ladite durée de vie.

14. Dispositif implantable selon l'une quelconque des revendications 10 à 13, comprenant en outre un amplificateur (304) pour amplifier lesdits signaux de sortie envoyés dudit au moins un photodétecteur (104) audit dispositif de détermination de constituants sanguins (301).

15. Dispositif implantable selon l'une quelconque des revendications précédentes 10 à 14, dans lequel un filtre optique (105) est agencé au niveau dudit photodétecteur (104) pour transmettre de manière sélective ladite lumière émise par lesdites molécules photoluminescentes (103) vers ledit au moins un photodétecteur (104).

**Fig. 1**

**Fig. 2**

**Fig. 3**

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4815469 A
- US 4476870 A
- US 20040152187 A